# EUROPÄISCHE PATENTSCHRIFT

(11) **EP 1 013 678 B1**
(45) Veröffentlichungstag und Bekanntmachung des Hinweises auf die Patenterteilung: **20.03.2002**
(21) Anmeldenummer: 99124190.2
(22) Anmeldetag: 03.12.1999
(51) Int. Cl.: C08F 210/00, C08F 10/00

(54) **Verfahren zum Verdichten ethylenisch ungesättigter Monomerer**
Process for compressing ethylenically unsatureted monomers
Procédé pour comprimer les monomères éthylèniquement insaturés

(30) Priorität: 22.12.1998 DE 19859391
(43) Veröffentlichungstag der Anmeldung: 28.06.2000
(73) Patentinhaber: BASF AKTIENGESELLSCHAFT, 67056 Ludwigshafen (DE)
(72) Erfinder: Sutoris, Heinz Friedrich, Dr., 67551 Worms (DE); Deckers, Andreas, Dr., 55234 Flomborn (DE); Weber, Wilhelm, Dr., 67435 Neustadt (DE)

(56) Entgegenhaltungen:
- EP-A- 0 811 590
- EP-A- 0 991 733
- US-A- 5 290 888

## Beschreibung

Gegenstand der vorliegenden Erfindung ist ein Verfahren zum Verdichten von ethylenisch ungesättigten Monomeren auf einen Druck zwischen 200 und 5000 bar in Abwesenheit eines Polymerisationsinitiators, dadurch gekennzeichnet, daß die Verdichtung in Gegenwart von Nitroxylverbindungen der Formel I 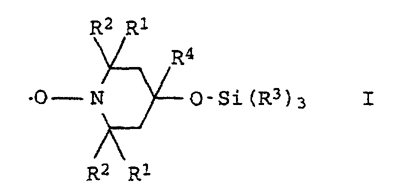 vorgenommen wird, wobei
- R¹, R²: C₁-C₄-Alkyl oder gemeinsam mit dem C-Atom, an das sie gebunden sind, einen 5- oder 6-gliedrigen gesättigten Kohlenwasserstoffring,
- R³: C₁-C₄-Alkyl und
- R⁴: Wasserstoff oder C₁-C₁₂-Alkyl bedeuten.

Weiterhin sind Gegenstand der vorliegenden Erfindung ein Verfahren zur Herstellung von Copolymeren durch entsprechende Verdichtung und anschließende Polymerisation und Copolymere erhältlich nach diesem Verfahren.

Derivate sterisch gehinderter Amine sind als Stabilisatoren von Kunststoffen und von radikalisch polymerisierbaren Monomeren seit langem bekannt.

Aus EP-A-178 168 ist ein Verfahren zur Inhibierung von α,β-ethylenisch ungesättigten Monocarbonsäuren, beispielsweise von Acrylsäure, bei deren Aufarbeitung durch Destillation bekannt.

In US-5 449 724 wird ein Verfahren zur Herstellung thermoplastischer Ethylenhomo- und -copolymerer bei Temperaturen von 40 bis 500°C und einem Druck zwischen 500 und 5000 bar in Gegenwart eines Radikalbildners und einer stabilen freien Radikalverbindung beschrieben. Die Anwesenheit der stabilen freien Radikalverbindung, insbesondere von Derivaten des 2,2,6,6-Tetramethylpiperidin-N-oxyls, führt dabei zu einer besonders engen Molekulargewichtsverteilung und damit verbunden zu besonderen physikalischen Eigenschaften der Polymere.

Aus der Schrift EP 0 811 590 ist ein Verfahren zum Verdichten von ethylenisch ungesättigten Monomeren auf Drücke zwischen 500 und 5000 bar unter Zugabe von Polymerisationsinhibitoren bekannt. Als solche werden u.a. auch Nitroxylverbindungen beschrieben.

Die in dieser Schrift gezeigten Beispiele 1 bis 3 belegen die vorteilhafte Wirkung verschiedener Mengen von N,N'-Bis-(2,2,6,6-tetramethylpiperidin-l-oxyl-4-yl)-N,N'-bisformyl-hexamethylendiamin im Hinblick auf die Inhibierung der vorzeitigen Polymerisation eines Ethylen/Acrylsäure-Gemischs.

So ermöglicht die Zugabe des genannten Inhibitors Laufzeiten des Nachverdichters von mindestens 124 h verglichen mit einer Laufzeit von lediglich 27 h bei Abwesenheit des Inhibitors. Die längeren Laufzeiten sind dabei aufgrund der deutlich geringeren Bildung von Polymerisaten möglich, da letztere zu Belägen und damit zu Undichtigkeiten im Nachverdichter führen und die Abschaltung und Reinigung dieses Aggregats notwendig machen.

Generell beobachtet man bei der Verdichtung von ethylenisch ungesättigten Monomeren auf solche Drücke zwischen 500 und 5000 bar, insbesondere bei der Verdichtung von Monomerengemischen aus Ethylen und Acrylsäure bzw. Acrylsäurederivaten, häufig schon vor der (erwünschten) Polymerisation in den Verdichtern und Vorverdichtern unerwünschte, vorzeitige Polymerisationen, die zu Belagbildungen führen und ein regelmäßiges Reinigen der Verdichter in kurzen Abständen erforderlich machen.

Übliche Inhibitoren, wie Methylhydrochinon und Hydrochinon, zeigen nur geringe Inhibitionswirkung und müssen in hohen Konzentrationen zugegeben werden. Nitroxylverbindungen, wie sie aus der Schrift EP 0 811 590 bekannt sind, zeigen eine deutlich bessere Wirkung, doch besteht ein permanenter Bedarf, noch wirksamere Inhibitoren zur Verfügung zu stellen.

Aufgabe der vorliegenden Erfindung war es daher, ein Verfahren zur Verdichtung von ethylenisch ungesättigten Monomeren zu finden, welches die aufgrund vorzeitiger Polymerisation stattfindende Belagbildung bei der Verdichtung noch effektiver als bisher vermindert.

Demgemäß wurde ein Verfahren zum Verdichten von ethylenisch ungesättigten Monomeren auf einen Druck zwischen 200 und 5000 bar in Abwesenheit eines Polymerisationsinitiators gefunden, welches dadurch gekennzeichnet ist, daß die Verdichtung in Gegenwart von Nitroxylverbindungen der eingangs gezeigten Formel I vorgenommen wird.

Nach dem erfindungsgemäßen Verfahren können alle üblichen ethylenisch ungesättigten Monomeren verdichtet werden. Als Monomere kommen beispielsweise Ethylen, Propylen, Buten und Butadien sowie Vinylester von C₂-C₁₈-Alkancarbonsäuren wie Vinylacetat und Vinylpropionat, C₂-C₁₈-Alkylester von Acryl- bzw. Methacrylsäure wie Methyl-, Ethyl-, Propyl-, Butyl- und 2-Ethylhexylacrylat und -methacrylat, Ester monoethylenisch ungesättigter Dicarbonsäuren wie Mono- und Diester der Malein- und Fumarsäure, monoethylenisch ungesättigte Carbonsäuren wie Acrylsäure, Methacrylsäure, Maleinsäure und Fumarsäure, Amide monoethylenisch ungesättigter Carbonsäuren wie Acrylamid, Methacrylamid, N-Mono- (C₁-C₁₈)-alkylacrylamid, N-Mono-(C₁-C₁₈)-alkylmethacrylamid, N-Di-(C₁-C₁₈)-alkylacrylamid und N-Di-(C₁-C₁₈)-alkylmethacrylamid, monoethylenisch ungesättigte Alkohole, C₁-C₄-Alkylvinylether und N-vinylheterocyclische Verbindungen wie N-Vinylpyrrolidon, N-Vinylcaprolactam und N-Vinylimidazole sowie N-Vinylformamid in Betracht.

Als Monomerengemische kommen insbesondere solche in Betracht, die Ethylen, Propylen, Buten und/oder Butadien und gegebenenfalls ein oder mehrere der oben genannten Comonomere enthalten. Besonders geeignet ist das erfindungsgemäße Verfahren zur Verdichtung von Monomerengemischen, wie sie zur Copolymerisation eingesetzt werden.

Insbesondere sind als Monomere/Comonomere auch Acrylsäure und/oder Methacrylsäure und/oder Derivate dieser Säuren zu nennen.

Geeignete Derivate dieser Säuren sind beispielsweise die C₁-C₁₈-Alkylester, C₁-C₁₈-Mono- und Dialkylamide sowie die unsubstituierten Amide. Dabei kommen als C₁-C₁₈-Alkylgruppen z.B. Methyl, Ethyl, n-Propyl, Isopropyl, n-Butyl, Isobutyl, sec-Butyl, tert-Butyl, n-Pentyl, Isopentyl, sec-Pentyl, tert-Pentyl, Neopentyl sowie die verschiedenen isomeren Hexyl-, Heptyl-, Octyl-, Nonyl-, Decyl-, Undecyl-, Dodecyl-, Tridecyl-, Tetradecyl-, Pentadecyl-, Hexadecyl-, Heptadecyl- und Octadecylreste in Betracht.

Insbesondere lassen sich nach dem erfindungsgemäßen Verfahren Gemische aus Ethylen und Acrylsäure oder Methacrylsäure verdichten.

Als C₁-C₄-Alkylgruppen für R¹, R² und R³ in Formel I kommen in Frage z.B. Methyl, Ethyl, n-Propyl, i-Propyl, n-Butyl, i-Butyl, sec-Butyl oder tert-Butyl.

Bevorzugt stehen die Reste R¹ und R² für gleiches C₁-C₄-Alkyl. Besonders bevorzugt handelt es sich bei den Resten R¹ und R² um Methylgruppen.

Die Reste R¹ und R² können auch gemeinsam mit dem C-Atom, an das sie gebunden sind, einen 5- oder 6-gliedrigen gesättigten Kohlenwasserstoffring bilden. So können R¹ und R² zusammen beispielsweise für eine Tetra- oder Pentamethylengruppe stehen.

Die Reste R³ können unterschiedlich sein, vorzugsweise bedeuten sie jedoch gleiches C₁-C₄-Alkyl. Bevorzugt handelt es sich bei R³ um gleiche Reste tert-Butyl oder i-Propyl, besonders bevorzugt um gleiche Reste Methyl.

Als C₁-C₁₂-Alkylgruppen für R⁴ kommen beispielsweise die oben genannten C₁-C₄-Alkylgruppen sowie Pentyl, sec-Pentyl, tert-Pentyl, Neopentyl, Hexyl, 2-Methylpentyl, Heptyl, 2-Methylhexyl, Octyl, Isooctyl, 2-Ethylhexyl, Nonyl, 2-Methylnonyl, Isononyl, 2-Methyloctyl, Decyl, Isodecyl, 2-Methylnonyl, Undecyl, Isoundecyl, Dodecyl und Isododecyl (die Bezeichnungen Isooctyl, Isononyl und Isodecyl sind Trivialbezeichnungen und stammen von den nach der Oxosynthese erhaltenen Carbonylverbindungen ab; vgl. dazu Ullmann's Encyklopedia of Industrial Chemistry, 5th Edition, Vol. A1. Seiten 290-293, sowie Vol. A10, Seiten 284 und 285) in Betracht.

In dem erfindungsgemäßen Verfahren können neben den Nitroxylverbindungen der Formel I auch Costabilisatoren eingesetzt werden. Als solche kommen beispielsweise aromatische Nitro- oder Nitrosoverbindungen sowie Hydroxylamine in Betracht.

Als aromatische Nitroverbindungen können beispielsweise 1,3-Dinitrobenzol, 1,4-Dinitrobenzol, 2,6-Dinitro-4-methylphenol, 2-Nitro-4-methylphenol, 2,4,6-Trinitrophenol, 2,4-Dinitro-1-naphthol, 2,4-Dinitro-6-methylphenol, 2,4-Dinitrochlorbenzol, 2,4-Dinitrophenol, 2,4-Dinitro-6-sec-butylphenol, 4-Cyano-2-nitrophenol, 3-Iodo-4-cyano-5-nitrophenol, besonders bevorzugt 2,6-Dinitro-4-methylphenol, 2-Nitro-4-methylphenol, 2,4-Dinitro-6-sec-butylphenol bzw. 2,4-Dinitro-6-methylphenol verwendet werden.

Als aromatische Nitrosoverbindungen kommen z.B. p-Nitrosophenol, p-Nitroso-o-kresol und p-Nitroso-N,N'-diethylanilin in Betracht.

Weiterhin kommen als Costabilisatoren Verbindungen aus der Gruppe der Chinone, der Phenothiazine und der Phenole, auch in Verbindung mit den zuvor genannten aromatischen Nitro- oder Nitrosoverbindungen sowie Hydroxylaminen, in Betracht.

Geeignete substituierte Phenole sind beispielsweise 4-tert-Butylbrenzcatechin, Methoxyhydrochinon, 2,6-Di-tert-butyl-4-methylphenol, n-Octadecyl-β-(3,5-di-tert-butyl-4-hydroxyphenyl)-propionat, 1,1,3-Tris-(2-methyl-4-hydroxy-5-tert-butylphenyl)-butan, 1,3,5-Trimethyl-2,4,6-tris-(3,5-di-tert-butyl-4-hydroxybenzyl)-benzol, 1,3,5-Tris-(3,5-di-tert-butyl-4-hydroxybenzyl)-iso-cyanurat, 1,3,5-Tris-[β-(3,5-di-tert-butyl-4-hydroxyphenyl)-propionyloxyethyl-isocyanurat, 1,3,5-Tris-(2,6-dimethyl-3-hydroxy-4-tert-butylbenzyl)-isocyanurat und Pentaerythrit-tetrakis-[β-(3,5-di-tert-butyl-4-hydroxy-phenyl)-propionat].

Die Nitroxylverbindungen der Formel I werden im erfindungsgemäßen Verdichtungsverfahren in einer Konzentration zwischen 0,00001 und 1 Gew.-%, bezogen auf die Menge der zu verdichtenden Monomeren, bevorzugt zwischen 0,0001 und 0,1 Gew.-% eingesetzt. Dieser Konzentrationsbereich gilt auch für die genannten Costabilisatoren.

Nach dem erfindungsgemäßen Verfahren werden die Monomeren auf einen Druck von 200 bis 5000 bar verdichtet. Die Verdichtung erfolgt vorzugsweise stufenweise, der Enddruck liegt vorzugsweise zwischen 1000 und 4000, besonders bevorzugt zwischen 1500 und 3000 bar.

Die Temperaturen bei der Verdichtung liegen vorzugsweise zwischen 20 und 140°C, besonders bevorzugt zwischen 30 und 100°C.

Während des Verdichtungsprozesses ist erfindungsgemäß kein Polymerisationsinitiator zugegen. Unter Polymerisationsinitiatoren sind dabei alle Verbindungen zu verstehen, die zur Initiierung der radikalischen Polymerisation den Monomeren zugesetzt werden, wie Azoverbindungen, organische Peroxide und Hydroperoxide. Eventuell im Verdichtungsgemisch anwesender Sauerstoff soll hier nicht als Polymerisationsinitiator verstanden werden.

Das erfindungsgemäße Verdichtungsverfahren ist vorzugsweise Teil eines Hochdruckcopolymerisationsverfahrens, welches dadurch gekennzeichnet ist, daß man die Comonomeren einzeln oder gemischt nach den erfindungsgemäßen Verfahren verdichtet und anschließend bei einer Temperatur zwischen 50 und 350°C durch Zugabe eines Polymerisationsinitiators polymerisiert.

Die bevorzugte Polymerisationstemperatur liegt zwischen 150 und 300°C, der bevorzugte Druck zwischen 1000 und 4000 bar, besonders bevorzugt zwischen 1500 und 3000 bar.

Die Polymerisation kann nach üblichen Methoden z.B. in Rohrreaktoren oder in Autoklavenreaktoren erfolgen. Es können alle üblichen Zusätze, beispielsweise Molekulargewichtsregler, Lösungsmittel usw., im Polymerisationsgemisch vorhanden sein.

Der Vorteil dieses erfindungsgemäßen Polymerisationsverfahrens liegt nicht nur in den längeren Laufzeiten der Verdichtungsapparaturen, sondern auch in einer Verbesserung der Polymerisationsprodukte. So neigt beispielsweise bei der Copolymerisation von Ethylen und Acrylsäure besonders die Acrylsäure zu vorzeitiger Polymerisation bei der Verdichtung. Dies führt zu Anteilen an Acrylsäurehomopolymeren oder zumindest von Homopolymerbereichen in den Copolymeren und damit zu beeinträchtigter Homogenität des Produktes und schlechterer Reproduzierbarkeit des Herstellverfahrens. Die nach dem erfindungsgemäßen Verfahren erhältlichen Copolymere weisen dagegen eine größere Homogenität auf.

### Beispiele 1 - 3:

Die Versuchsführung erfolgte analog zu jener in den Beispielen 1 bis 3 der Schrift EP 0 811 590. Anstelle der Verbindung N,N'-Bis-(2,2,6,6-tetramethylpiperidin-1-oxyl-4-yl)-N,N'-bisformylhexamethylendiamin wurde jedoch 1-Oxyl-4-trimethylsiloxy-2,2,6,6-tetramethylpiperidin ("TMS-TEMPO") verwendet.

In einem Vorverdichter wurde Ethylen auf einen Druck von 220 bar komprimiert. In den komprimierten Gasstrom (1400 kg/Stunde) wurden in 60 Minuten 85 l eines Gemisches aus Acrylsäure und Isododecan (1:1, Vol:Vol), in welchem die in der Tabelle angegebenen Mengen TMS-TEMPO gelöst worden waren, gepumpt. Die Mischung wurde im Nachverdichter auf 2300 bar verdichtet und kontinuierlich in einen 35 l Stahldurchflußautoklaven überführt. Die Polymerisation wurde durch Zugabe von 0,0025 mol-% (bezogen auf die Gesamtmolmenge der Monomeren) tert-Butylperpivalat gestartet. Die Reaktionstemperatur betrug 220°C. Die Reaktion wurde beendet, sobald die auf Ablagerungen zurückzuführende Leckgasmenge am Nachverdichter 50 kg/Stunde überschritt.

Die Ergebnisse der Versuche zeigt die folgende Tabelle (in Klammern sind die Daten für den in EP 0 811 590 verwendeten Inhibitor angegeben):

| Beispiel | Inhibitorkonz. [Gew.-%] | Laufzeit [h] |
|---|---|---|
| 1 | 0,020 | >168 (> 168) |
| 2 | 0,010 | >168 (> 168) |
| 3 | 0,005 | >168 (124) |
| Vergleich | 0 | 25 (27) |

Bei Verwendung von TMS-TEMPO erhält man bereits ab Konzentrationen von 0,005 Gew.-% Laufzeiten von über einer Woche. Demgegenüber muß der herkömmliche Inhibitor in Mengen bis zum doppelten dieser Konzentration zugegeben werden, um denselben Effekt zu zeigen.

## Patentansprüche

1. Verfahren zum Verdichten von ethylenisch ungesättigten Monomeren auf einen Druck zwischen 200 und 5000 bar in Abwesenheit eines Polymerisationsinitiators, **dadurch gekennzeichnet, daß** die Verdichtung in Gegenwart von Nitroxylverbindungen der Formel I 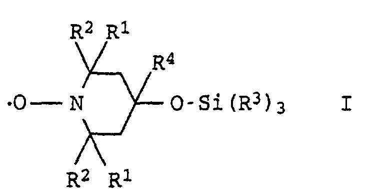 vorgenommen wird wobei
R¹, R² C₁-C₄-Alkyl oder gemeinsam mit dem C-Atom, an das sie gebunden sind, einen 5- oder 6-gliedrigen gesättigten Kohlenwasserstoffring,
R³ C₁-C₄-Alkyl und
R⁴ Wasserstoff oder C₁-C₁₂-Alkyl.

2. Verfahren nach Anspruch 1, **dadurch gekennzeichnet, daß** als ethylenisch ungesättigte Monomere Gemische, enthaltend Ethylen, Propylen, Buten und/oder Butadien, eingesetzt werden.

3. Verfahren nach Anspruch 1, **dadurch gekennzeichnet, daß** als ethylenisch ungesättigte Monomere Gemische, enthaltend Acrylsäure und/oder Methacrylsäure und/oder Derivate dieser Säuren, eingesetzt werden.

4. Verfahren nach Anspruch 1, **dadurch gekennzeichnet, daß** als ethylenisch ungesättigte Monomere Gemische, enthaltend Ethylen und Acrylsäure oder Methacrylsäure, eingesetzt werden.

5. Verfahren nach den Ansprüchen 1 bis 4, **dadurch gekennzeichnet, daß** die Verdichtung in Anwesenheit von Hydroxylaminen oder von aromatischen Nitro- oder Nitrosoverbindungen vorgenommen wird.

6. Verfahren nach den Ansprüchen 1 bis 5, **dadurch gekennzeichnet, daß** die Verdichtung in Anwesenheit einer Verbindung aus der Gruppe der Chinone, der Phenothiazine und der Phenole vorgenommen wird.

7. Verfahren zur Herstellung von Copolymeren, **dadurch gekennzeichnet, daß** man die Comonomeren einzeln oder gemischt nach einem Verfahren gemäß den Ansprüchen 1 bis 6 verdichtet und anschließend bei einer Temperatur zwischen 50 und 350°C durch Zugabe eines Polymerisationsinitiators polymerisiert.

8. Copolymere erhältlich nach einem Verfahren gemäß Anspruch 7.

## Claims

1. A process for pressurizing ethylenically unsaturated monomers to 200 - 5000 bar in the absence of a polymerization initiator, which comprises effecting said pressurizing in the presence of nitroxyl compounds of the formula I 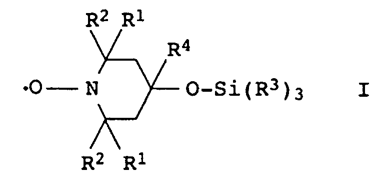 where
R¹ and R² are singly C₁-C₄-alkyl or combine with the joining carbon atom to form a 5- or 6-membered saturated hydrocarbon ring,
R³ is C₁-C₄-alkyl, and
R⁴ is hydrogen or C₁-C₁₂-alkyl.

2. A process as claimed in claim 1, wherein said ethylenically unsaturated monomers are mixtures comprising ethylene, propylene, butene and/or butadiene.

3. A process as claimed in claim 1, wherein said ethylenically unsaturated monomers are mixtures comprising acrylic acid and/or methacrylic acid and/or derivatives thereof.

4. A process as claimed in claim 1, wherein said ethylenically unsaturated monomers are mixtures comprising ethylene and acrylic acid or methacrylic acid.

5. A process as claimed in any of claims 1 to 4, wherein said pressurizing is effected in the presence of hydroxylamines or of aromatic nitro or nitroso compounds.

6. A process as claimed in any of claims 1 to 5, wherein said pressurizing is effected in the presence of a compound selected from the group consisting of the quinones, the phenothiazines and the phenols.

7. A process for preparing copolymers, which comprises employing a process as claimed in any of claims 1 to 6 to pressurize the comonomers individually or mixed and then polymerizing at from 50 to 350°C by adding a polymerization initiator.

8. Copolymers obtainable by a process as claimed in claim 7.

## Revendications

1. Procédé pour la compression de monomères éthyléniquement insaturés à une pression entre 200 et 5000 bars en l'absence d'un initiateur de polymérisation , **caractérisé en ce que** la compression est effectuée en présence de combinaisons nitroxyle de la formule I 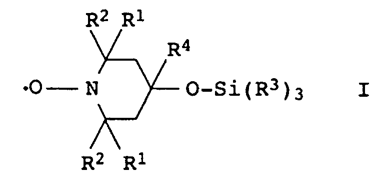 dans laquelle:
R¹, R² sont un alkyle en C₁ et C₄ ou constituent, conjointement avec l'atome de C auquel ils sont liés, un cycle hydrocarbure saturé à 5 ou 6 chaînons.
R³ est un alkyle en C₁ - C₄ et
R⁴ est l'hydrogène ou un alkyle en C₁-C₁₂.

2. Procédé selon la revendication 1, **caractérisé en ce que** on utilise, comme monomères éthyléniquement insaturés, des mélanges contenant l'éthylène, le propylène, le butène et/ou le butadiène.

3. Procédé selon la revendication 1, **caractérisé en ce que** l'on utilise, comme monomères éthyléniquement insaturés, des mélanges contenant l'acide acrylique et/ou l'acide méthacrylique et/ou des dérivés de ces acides.

4. Procédé selon la revendication 1, **caractérisé en ce que** l'on utilise, comme monomères éthyléniquement insaturés, des mélanges contenant l'éthylène et l'acide acrylique ou l'acide méthacrylique.

5. Procédé selon les revendications 1 à 4, **caractérisé en ce que** la compression est effectuée en présence d'hydroxylamines ou de combinaisons nitro ou nitrosoaromatiques.

6. Procédé selon les revendication 1 à 5, **caractérisé en ce que** la compression est effectuée en présence d'une combinaison issue du groupe des quinones, des phénothiazines et des phénols.

7. Procédé pour la préparation de copolymères, **caractérisé en ce que** l'on comprime les comonomères, individuellement ou mélangés, par un procédé suivant les-revendications 1 à 6 et qu'on les polymérise ensuite à une température entre 50 et 350°C par addition d'un initiateur de polymérisation.

8. Copolymères pouvant être obtenus par un procédé selon la revendication 7.
